# EUROPÄISCHE PATENTANMELDUNG

(11) **EP 1 570 813 A1**
(43) Veröffentlichungstag der Anmeldung: **07.09.2005**
(21) Anmeldenummer: 04005341.5
(22) Anmeldetag: 05.03.2004
(51) Int. Cl.: A61F 2/44, A61B 17/16

(54) **Zervikale Bandscheibenprothese mit Luxationssicherung und Instrumentarium**

(71) Anmelder: Cervitech, Inc., Rockaway, NJ 07866 (US)
(72) Erfinder: Link, Helmut D., 22397 Hamburg (DE); Keller, Arnold, 23863 Kayhude (DE)
(74) Vertreter: Glawe, Delfs, Moll

(57) **Zusammenfassung**

Eine zervikale Zwischenwirbelprothese umfasst eine untere und eine obere Verankerungsplatte (11, 12) mit dazwischen angeordnetem Prothesenkern (10) zur gelenkigen Verbindung. Die Verankerungsplatten (11, 12) sind mit ihrer Verankerungsplattenoberfläche zur Anlage an benachbarte Wirbelkörper ausgebildet. Die Erfindung sieht vor, dass mindestens eine Verankerungsplattenoberfläche (11, 12) einen rippenartigen Vorsprung (18) aufweist, mit dem formschlüssig in den Wirbelkörper eingegriffen wird. Zur Herstellung einer entsprechenden Ausnehmung in dem Wirbelkörper ist ein Instrument mit einem Griff, einem Stiel und einem Kopfstück vorgesehen, das in dem Kopfstück ein versenkbares Räumelement aufweist. Damit wird eine wesentlich verbesserte Sicherung der zervikalen Zwischenwirbelprothese gegenüber eine unbeabsichtigten Bewegung erreicht. Der am hinteren Rand der Wirbelsäule laufende Markkanal ist damit vor Beeinträchtigungen geschützt.

## Beschreibung

Die Erfindung betrifft eine zervikale Zwischenwirbelprothese umfassend eine untere und eine obere Verankerungsplatte, die mit jeweils einer Verankerungsplattenoberfläche zur Anlage an einem benachbarten Wirbelkörper ausgebildet sind, sowie einen dazwischen angeordneten Prothesenkern, der eine gelenkige Verbindung zwischen den Verankerungsplatten bildet.

Zwischenwirbelprothesen, die zur Implantation im zervikalen Bereich der Wirbelsäule vorgesehen sind, müssen aufgrund der geringen Abmessungen der Wirbelsäule in diesem Bereich äußerst genau positioniert sein. Die Prothese darf sich nach der Implantation in ihrer Knochenverankerung nicht unbeabsichtigt verschieben. Schon bei einer geringen Verschiebung von Prothesenteilen nach dorsal besteht die Gefahr, dass Rückenmarksnerven betroffen sind. Es ist daher von großer Wichtigkeit, die Zwischenwirbelprothese an ihrem implantierten Ort sicher zu fixieren. Gerade im Bereich der Halswirbelsäule ist dies aber schwierig, da aufgrund der geringen Abmessungen nur wenig Raum zur Verfügung steht.

Es ist bekannt (WO-A-030 75 803), die Verankerungsplatten der Zwischenwirbelprothesen an ihrem ventralen Rand mit einem Flansch zu versehen und diesen mittels Schrauben an dem Wirbelkörper zu befestigen. Um eine ausreichende Verbindungssicherheit zu haben, müssen die Schrauben und der Flansch Abmessungen aufweisen, die sich mit den schwierigen Implantationsbedingungen im Bereich der Halswirbelsäule nur schwer vereinbaren lassen. Diese Schwierigkeit wird bei einer anderen Konstruktion umgangen (WO-A-030 75 804), die einen verkürzten Flansch ohne Schraubbefestigung zur Sicherung gegenüber einem Verrutschen nach dorsal und eine gezahnte Oberfläche der Verankerungsplatten als Sicherung gegenüber einem Verrutschen nach ventral vorsieht. Diese Konstruktion eignet sich gut zur Implantation unter den beengten Bedingungen im Bereich der Halswirbelsäule. Unter gewissen Bedingungen ist dabei eine Erhöhung der Verbindungssicherheit wünschenswert.

Es ist weiter bekannt, zusätzlich zu der Zahnung in anterior-posterior (AP-)Richtung verlaufende, selbstschneidende Rippen vorzusehen (WO 03/075804). Diese drücken sich selbsttätig in die Endfläche des Wirbelkörpers ein. Eine Sicherung gegenüber unerwünschter Bewegung in AP-Richtung wird damit nicht erreicht. Aufgrund der selbstschneidenden Eigenschaft werden von der Rippe beim Einsetzen erhebliche Kräfte in die Verankerungsplatten eingeleitet, die teilweise auch in horizontaler Richtung wirken. Dadurch steigt das Risiko einer Fehlpositionierung.

Der Erfindung liegt die Aufgabe zu Grunde, die Befestigungssicherheit einer zervikalen Zwischenwirbelprothese bei Beibehaltung guter Implantierbarkeit zu erhöhen.

Die erfindungsgemäße Lösung liegt in einer zervikalen Zwischenwirbelprothese mit den Merkmalen des Anspruchs 1. Sie liegt weiter in einem Instrument zur Implantation einer solchen zervikalen Zwischenwirbelprothese gemäß Anspruch 7. Vorteilhafte Weiterbildungen sind Gegenstand der abhängigen Ansprüche.

Erfindungsgemäß ist bei einer zervikalen Zwischenwirbelprothese umfassend eine untere und eine obere Verankerungsplatte, die mit jeweils einer Verankerungsplattenoberfläche zur Anlage an einem benachbarten Wirbelkörper ausgebildet sind, sowie einen dazwischen angeordneten Prothesenkern, der eine gelenkige Verbindung zwischen den Verankerungsplatten bildet, vorgesehen, dass mindestens eine der beiden Verankerungsplattenoberflächen zum Wirbelkörper einen rippenartigen Vorsprung zum formschlüssigen Eingriff in den Wirbelkörper aufweist.

Die Erfindung beruht auf dem Gedanken, mittels des erfindungsgemäßen rippenartigen Vorsprungs eine formschlüssige Halterung der Grund- oder Verankerungsplatte am Wirbelkörper zu erreichen. Der in den Wirbelkörper fassende Vorsprung verhindert eine unerwünschte Verschiebung der Verankerungsplatte gegenüber dem Wirbelkörper. Einen Disloziierung der zervikalen Zwischenwirbelprothese als Ganzes kann dadurch vermieden werden. Es hat sich gezeigt, dass mit dem formschlüssig in den Wirbelkörper eingreifenden Vorsprung eine so feste Verankerung der Zwischenwirbelprothese an dem Wirbelkörper erreicht wird, dass eine Subluxation im Sinne eines Auswanderns nach dorsal nicht zu befürchten ist. Es versteht sich, dass der Vorsprung nicht nur eine unerwünschte Bewegung in die dorsale Richtung verhindern kann, sondern auch in die entgegengesetzte Richtung nach ventral. Die Betriebssicherheit der mit dem erfindungsgemäßen Vorsprung versehenen Zwischenwirbelprothese erhöht sich dadurch beträchtlich.

Die Form des Vorsprungs ist an sich beliebig. Wichtig ist vor allem, dass in der AP-Richtung verlaufenden Einsetzrichtung der Zwischenwirbelprothese in den Raum zwischen den benachbarten Wirbelkörpern der formschlüssige Eingriff des Vorsprungs in den Wirbelkörper als Hinterschnitt ausgeführt ist. Es hat sich bewährt, den Vorsprung als Rippe auszuführen. Vorzugsweise ist sie in einer Ebene parallel zum ventral und dorsalen Rand der Verankerungsplatte angeordnet. Im implantierten Zustand steht damit die Rippe quer zur AP-Richtung, womit sie einer unerwünschten Verschiebung größtmöglichen Widerstand entgegensetzt. Es kann zweckmäßig sein, die Oberkante der Rippe konvex auszuführen. Bewährt hat sich ein Krümmungsdurchmesser von 3 bis 10 mm. Das Einsetzen der Verankerungsplatte mit ihrem Vorsprung in ihre Position an dem Wirbelkörper vereinfacht sich dadurch, weil die Verankerungsplatte sich aufgrund der konvexen Formgebung in ihre Position einsuchen kann. Außerdem hat die konvex gerundete Gestaltung den Vorteil, dass in den Wirbelkörper ragende Ecken vermieden werden. Der Gefahr unerwünschter Spitzenbelastungen im Eckbereich wird damit entgegengewirkt. Schließlich hat die konvexe, idealerweise kreisabschnittsförmige Gestaltung der Rippe auch den Vorteil, dass mit einem formentsprechenden rotierbaren Schneidwerkzeug einfach die kongruente Ausnehmung im Wirbelkörper hergestellt werden kann.

Bei einer anderen zweckmäßigen Ausführungsform ist der Vorsprung in zwei oder mehr Segmente mit dazwischen liegender Lücke unterteilt. Vorzugsweise sind sie bolzenartig ausgeführt. Mit einer solchen bolzenartigen Ausformung des Vorsprungs kann eine Aufnahme zum formschlüssigen Eingriff in den Wirbelkörper leicht durch einfaches Bohren hergestellt werden. Jedoch ist es bei mehreren Segmenten nicht unbedingt erforderlich, dass jedes in eine eigene Ausnehmung greift. Genauso gut kann als Ausnehmung für den formschlüssigen Eingriff ein Schlitz vorgesehen sein, in den die in einer Ebene angeordneten Segmente eingreifen.

Vorzugsweise ist der Vorsprung außerhalb eines Randbereichs der Verankerungsplattenoberfläche angeordnet. Unter einem Randbereich wird der äußere Sektor der Verankerungsplattenoberflächen verstanden, der etwa 1/10 der gesamten Verankerungsplattenoberfläche einnimmt. In diesem Bereich ist die Gefahr eines Ausbrechens des Vorsprungs aus dem formschlüssigen Eingriff in den Wirbelkörper unter hoher Belastung verringert. Zwar ist der Wirbelkörper an seinen Randbereichen, insbesondere im ventralen und dorsalen Rand, von einer höheren Festigkeit als im dazwischen liegenden Bereich. Ein Anordnen des Vorsprungs im anterioren oder postserioren Bereich hätte aber den Nachteil, dass die Ausnehmung zur formschlüssigen Aufnahme des Vorsprungs in einem harten und spröden Bereich des Wirbelkörpers zu bilden wäre. Es bestünde die Gefahr, dass dabei Knochenteile absplittern. Es hat sich gezeigt, dass eine Anordnung des Vorsprungs etwas nach dorsal versetzt, vorzugsweise in einem Bereich zwischen 3/5 und 3/4 der Erstreckung in AP-Richtung, sowohl eine hohe Kraftübertragungsmöglichkeit bereitstellt wie auch sicher und ohne Gefahr von Absplitterungen zu implantieren ist.

Der Vorsprung kann eine Höhe von 0,3 bis 5,0 mm über dem Niveau der Verankerungsplattenoberfläche aufweisen. Ist diese mit einer Zahnung versehen, was zur weiteren Erhöhung der Verbindungssicherheit von Vorteil ist, so ist das Niveau der Verankerungsplattenoberfläche durch die Oberkante der Zahnung definiert. Mit Vorteil ist der Vorsprung nach oben hin verjüngend ausbildet. Damit kann eine selbstzentrierende Wirkung beim Einsetzen der Verankerungsplatte an den Wirbelkörper erreicht werden. Kleinere Ungenauigkeiten können auf diese Weise ausgeglichen werden. Die Implantation vereinfacht sich.

Ein erfindungsgemäßes Instrument zum Implantieren einer solchen zervikalen Zwischenwirbelprothese umfasst einen Griff, einen Stiel und ein Kopfstück an einem vom Griff entfernten Ende, dessen Abmessungen so gewählt sind, dass es in den zur Aufnahme der Zwischenwirbelprothese geschaffenen Raum einschiebbar ist, wobei das Kopfstück ein Räumelement zum Schaffen einer Ausnehmung in kranial-kaudaler Richtung aufweist und eine Betätigungseinrichtung für das Räumelement vorgesehen ist, das zwischen einer Ruheposition, in der es in dem Kopfstück versenkt ist, und einer Arbeitsposition, in der es quer zum Stiel aus dem Kopfstück herausragt, beweglich ist. Das Instrument kann mit seinem Kopfstück an die vorgesehene Implantationsstelle geschoben werden, die in an sich bekannter Weise präpariert wurde. Befindet sich das Räumelement in seiner Ruheposition, in der es in dem Kopfstück versenkt ist, so kann das Kopfstück ohne Schwierigkeiten an die vorgesehene Implantationsstelle vorgeschoben werden. Das Erreichen der richtigen Position wird zweckmäßigerweise durch Röntgenkontrollen verifiziert.

Dazu kann es zweckmäßig sein, gesonderte Röntgenmarken an dem Kopfstück vorzusehen. Zur Schaffung einer Ausnehmung an der Endfläche des Wirbelkörpers, in den der an der Verankerungsplatte der Zwischenwirbelprothese angeordnete rippenartige Vorsprung formschlüssig eingreifen kann, wird das Räumelement in seine Arbeitsposition bewegt. Durch Betätigen des Räumelements kann dann die Ausnehmung geschaffen werden. Zum Herausnehmen des Instruments kann dann das Räumelement wieder in seine versenkte Ruheposition gebracht werden. Das Räumelement kann einseitig oder beidseitig ausfahrbar sein. Die Erfindung schafft damit ein Instrument zur Implantation der erfindungsgemäßen Zwischenwirbelprothese, das leicht an die vorgesehene Implantationsstelle angeschoben werden kann und in dieser Position Ausnehmungen zur formschlüssigen Aufnahme der Vorsprünge in den Wirbelkörper schafft.

Das Kopfstück kann gleichzeitig als Räumnadel (Raspel mit Querzähnen) ausgeführt sein, wie sie zum Herstellen eines Implantatbettes durch Knochenabtragung in den Wirbelkörpern in anterior / posterior Richtung verwendet wird, oder auch als Probeprothese, mit der man unter Röntgenkontrolle die Größe und Position der später zu implantierenden Prothese abschätzt.

Zweckmäßigerweise ist das Räumelement eine Messerscheibe. Sie weist vorzugsweise mindestens ein Paar über den Umfang versetzt angeordnete Schneidfinnen auf. In der Ruheposition stehen die Schneidfinnen an einer solchen Position, dass sie nicht aus dem Kopfstück herausragen. Ist die Messerscheibe durch das Betätigungselement verdreht, ragen die Schneidfinnen aus dem Kopfstück senkrecht zur Richtung des Stiels aus den Kranial/Kaudal-Flächen des Kopfstücks heraus und greifen somit bei eingeschobenem Kopfstück in den benachbarten Wirbelkörper ein. Durch Bewegen der Messerscheibe wird dann die Ausnehmung geschaffen. Es ist zweckmäßig, die Schneidfinnen eines Paars genau gegenüberliegend auf der Messerscheibe anzuordnen. Dadurch kann erreicht werden, dass die Ausnehmung in beiden benachbarten Wirbelkörpern bei demselben Schneidvorgang herstellbar ist. Damit ist sichergestellt, dass die Ausnehmungen in einer Flucht liegen. Außerdem wirken dann in der Summe keine horizontalen Kräfte auf das Kopfstück ein. Es kann aber auch vorgesehen sein, die Schneidfinnen nicht genau gegenüberliegend anzuordnen, sondern um einen bestimmten Winkel zu versetzen, der so bemessen ist, dass beim Verdrehen der Messerscheibe aus ihrer Ruheposition zuerst eine Schneidfinne in Kontakt mit einem der beiden benachbarten Wirbelkörper kommt und eine Ausnehmung darin schneidet, und erst dann, wenn diese Schneidfinne sich in diesen Wirbelkörper eingearbeitet hat, die andere Schneidfinne aus der gegenüberliegenden kranialen/kaudalen Fläche des Kopfstücks austritt und in den anderen der beiden Wirbelkörper schneidet. Das hat den Vorteil, dass die zur Betätigung erforderlichen Kräfte geringer sind, da nicht gleichzeitig in beide Wirbelkörper geschnitten wird, sondern nacheinander.

Um ein leichteres Schneiden und ein Ausbrechen von Knochenmaterial zu vermeiden, ist es zweckmäßig, Schneidfinnen mit unterschiedlichen Höhen vorzusehen. Sie sind so angeordnet, dass beim Bewegen aus der Ruheposition zuerst die Schneidfinne mit der niedrigeren Höhe herausragt und in den Wirbelkörper schneidet und danach die Schneidfinne mit der größeren Höhe. Es versteht sich, dass auch mehr als zwei Schneidfinnen unterschiedlicher Höhe vorgesehen sein können. Um gleichzeitig in beide benachbarten Wirbelkörper schneiden zu können sind die Schneidfinnen in unterschiedlicher Höhe zweckmäßigerweise paarig gegenüberliegend angeordnet.

Es können aber auch andere Arten von Räumelementen vorgesehen sein. Bei einer anderen Ausführungsform ist es als Bohrer ausgebildet. Zweckmäßigerweise ist zur Betätigung ein Schub/Schraubtrieb vorgesehen. Es können auch mehrere Bohrer vorgesehen sein, zweckmäßigerweise sind mindestens zwei quer zum Stiel angeordnete Bohrer vorgesehen.

Vorzugsweise weist das Räumelement eine bewegliche Drehachse auf. Dies ermöglicht eine einfache Betätigung mittels einer drehbaren Welle.

Zweckmäßigerweise ist das Räumelement entlang einer Führung verschiebbar, so dass ein Schlitz gefräst werden kann.

Das Betätigungselement umfasst vorzugsweise einen Handgriff und eine Übertragungswelle. Eine solche manuelle Betätigung hält das Instrument einfach und ermöglicht es dem Operateur, das Räumelement nach seinem Gefühl zu bewegen. Es hat sich bewährt, das Betätigungselement mit einer Indexeinrichtung zu versehen, welche die Ruheposition markiert. Damit kann der Operateur sicherstellen, dass das Räumelement sich in seiner Ruheposition befindet, bevor er das Instrument einschiebt oder herauszieht. Eine manuelle Betätigung ist aber nicht zwingend, es kann ebenfalls eine Drehantriebskupplung für eine motorische Betätigung vorgesehen sein.

Die Erfindung wird nachfolgend unter Bezugnahme auf die Zeichnung erläutert, in der vorteilhafte Ausführungsbeispiele dargestellt sind. Es zeigen:
- Fig. 1: eine perspektivische Ansicht eines Ausführungsbeispiels einer erfindungsgemäßen Zervikalprothese;
- Fig. 2: eine Ansicht von posterior der Zervikalprothese gemäß Fig. 1;
- Fig. 3: eine posterior-kraniale Ansicht;
- Fig. 4: eine Ansicht des posterioren Endes eines Ausführungsbeispiels für ein erfindungsgemäßes Instrument;
- Fig. 5: eine Schnittansicht durch ein Kopfstück des Instruments gemäß Fig. 4;
- Fig. 6: eine Schnittansicht eines alternativen Ausführungsbeispiels für ein Instrument;
- Fig. 7: das erfindungsgemäße Instrument in einer perspektivischen Ansicht von schräg hinten;
- Fig. 8: eine vergrößerte Darstellung des Kopfstücks des Instruments;
- Fig. 9: eine Aufsicht auf das erfindungsgemäße Instrument;
- Fig. 10: eine schematische Ansicht zweier benachbarter Wirbelkörper, zwischen die eine erfindungsgemäße zervikalprothese zu implantieren ist;
- Fig. 11: eine schematische Ansicht der beiden Wirbelkörper gemäß Fig. 9 beim Vorbereiten der Implantationsstelle;
- Fig. 12: eine weitere Stufung der Vorbereitung gemäß Fig. 10; und
- Fig. 13: die zwischen die benachbarten Wirbel implantierte Zervikalprothese.

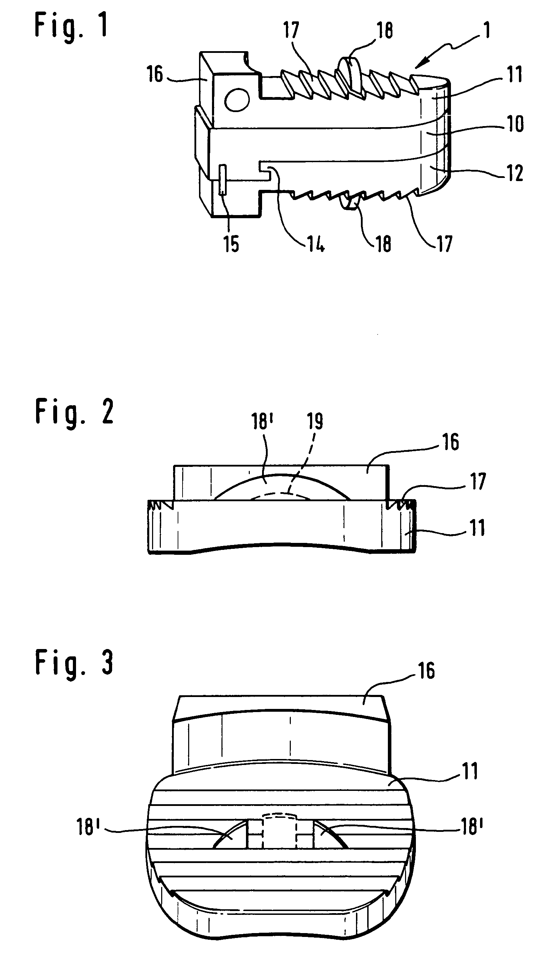

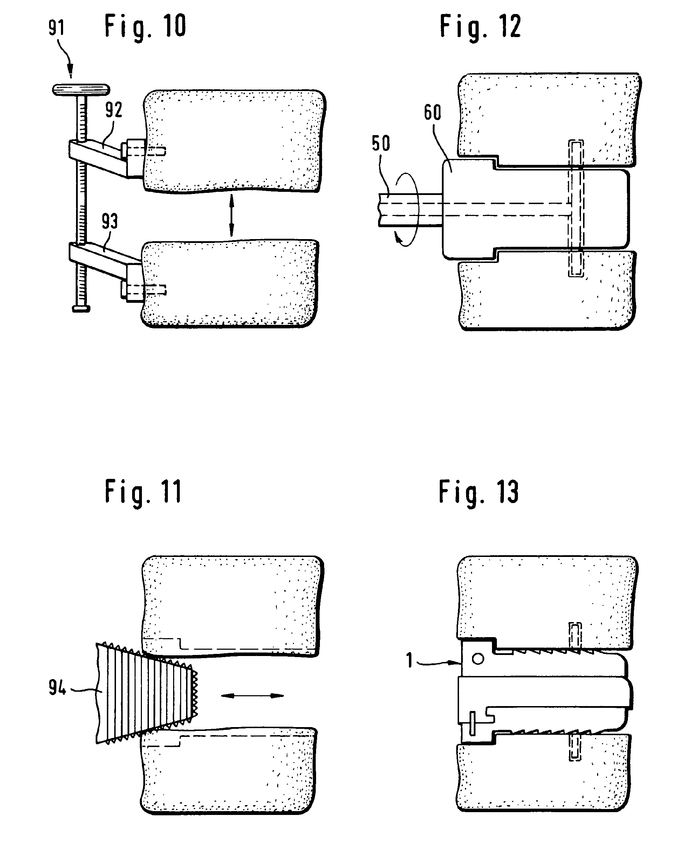

Bei dem in den Figuren 1 bis 3 dargestellten Ausführungsbeispiel handelt es sich um eine erfindungsgemäße Zervikalprothese, die in ihrer Gesamtheit mit dem Bezugszeichen 1 versehen ist. Sie ist zur Implantation in den Zwischenraum zweier benachbarter Wirbelkörper der Halswirbelsäule (siehe Fig. 13) vorgesehen.

Die Zervikalprothese 1 besteht aus einer oberen Abschlussplatte 11 und einer unteren Abschlussplatte 12 mit einem dazwischen angeordneten Gleitkern 10. Die Zervikalprothese 1 ist zur Implantation in den Zwischenraum zweier benachbarter Wirbel der Halswirbelsäule eines Menschen vorgesehen. Dabei wird die obere Verankerungsplatte 11 an der Unterseite des kranialen Wirbels und die untere Verankerungsplatte 12 an der Oberseite des kaudalen Wirbels befestigt. Die Verankerungsplatten 11, 12 bestehen aus hartem widerstandsfähigem Material, insbesondere Titan oder einem anderen biokompatiblen Material. Vorzugsweise sind zumindest die zur Anlage an die benachbarten Wirbelkörper dienenden Oberflächen der Verankerungsplatten 11, 12 mit einer knochenwachstumsfördernden Beschichtung versehen, beispielsweise Hydroxylapatit. Der Prothesenkern 10 besteht aus Polethylen oder einem anderen gleitgünstigen und hinreichend verschleißfesten Kunststoff. Der Prothesenkern 10 ist mit der unteren Verankerungsplatte 12 fest, wenn auch lösbar, verbunden. Zur Verbindung dient eine hinterschnittene Randleiste 14 an der anterioren Seite (in Fig. 1 links) der unteren Verankerungsplatte 12, in die der mit einer komplementären Nut versehene Prothesenkern eingeschoben werden kann. In seiner eingeschobenen Stellung wird der Prothesenkern 10 mittels eines Plättchens 15 gesichert. Die Oberseite des Prothesenkerns 10 und die untere Seite der oberen Verankerungsplatte 11 bilden zusammen wirkende, komplementäre Gleitflächen, die vorzugsweise sphärisch gestaltet sind.

Die Verankerungsplatten 11, 12 sind an ihrem anterioren Rand mit einem als Flansch ausgebildeten Rand versehen, der bei der oberen Verankerungsplatte 11 kranial und bei der unteren Verankerungsplatte 12 kaudal hervorragt. Die nach posterior (in Fig. 1 rechts) gewandte Rückseite des Flansches 16 weist eine Anschlagfläche für den ventralen Rand des Wirbelkörpers auf. Um ein Hervorstehen der Verankerungsplatten 11, 12 nach ventral und dadurch eventuell ausgelöste Irritationen innerer Organe zu vermeiden, ist der ventrale Rand der Wirbelkörper vorzugsweise so bearbeitet, dass eine Ausnehmung entsteht, in die der Flansch 16 der Verankerungsplatten 11, 12 versenkt ist. Vorzugsweise ist der anteriore Rand des Flansch 16 gerundet, um ein sicheres Anliegen des Flansches 16 an dem Wirbelkörper zu erreichen. Damit einher geht auch ein gewisser Schutz gegenüber unerwünschten seitlichen Verschiebungen. Damit zur versenkten Anordnung des Flansches 16 nicht zuviel Material von den Wirbelkörpern abgenommen werden muss, sind seine Abmessungen zweckmäßigerweise gering bemessen. Insbesondere soll er nur eine geringe Höhe über der Oberseite der Verankerungsplatte 11 bzw. unter der Unterkante der Verankerungsplatte 12 aufweisen. Er soll zwischen 0,5 und 2 mm, vorzugsweise zwischen 0,8 und 1,3 mm liegen. Relativ auf die Größe der Zwischenwirbelprothese ausgedrückt soll die Höhe etwa 0,5 bis 2/10 der Gesamtabmessung in der anterior/posterioren Richtung (AP-Richtung) messen.

Die Oberseiten der Verankerungsplatten 11, 12 sind über den größten Teil ihrer Fläche mit Zähnen 17 versehen. Sie sind sägezahnförmig ausgebildet, wobei die steilere Flanke nach anterior zum Flansch 16 hinweist und die weniger steile Flanke nach posterior weist. Die steile Flanke der Zähne 17 schließt vorzugsweise einen Winkel von 70 bis 90 Grad mit der Ebene der Verankerungsplatten 11, 12 ein. Die Zähne 17 sind so ausgebildet, dass sie quer zur AP-Richtung orientiert sind. Aufgrund dieser Orientierung üben die Zähne 17 eine nach posterior wirkende Kraft auf die Zwischenzervikalprothese 1 aus und verhindern damit eine unerwünschte Verschiebung der Zervikalprothese 1 nach anterior. Der Flansch 16 wiederum sichert die Zervikalprothese 1 gegenüber einer Bewegung nach posterior. Im Ergebnis ist damit die Zervikalprothese gegenüber einer unerwünschten Bewegung in beiden Richtungen gesichert.

Zur Verbesserung der Sicherungswirkung und zum Schutz gegenüber Subluxation ist an der Oberfläche der Verankerungsplatten 11, 12 jeweils eine nach kranial bzw. kaudal ragende Rippe angeordnet. Sie ist parallel zu den Zähnen 17 quer zur AP-Richtung orientiert. Ihre Oberseite ist als Kreisbogensegment ausgebildet. Die Stärke der Rippe 18 ist über die gesamte Höhe vorzugsweise konstant, sie kann sich aber nach oben hin auch verjüngen. Eine selbstschneidende Funktion ist nicht vorgesehen. Die Rippe 18 ist durch Schweißen oder Hartlöten fest mit der jeweiligen Verankerungsplatte 11, 12 verbunden. Es kann aber auch vorgesehen sein, dass die Verankerungsplatte 11, 12 und die Rippe 18 aus jeweils einem Stück hergestellt sind.

Ist der die Wirbelkörper zusammenhaltende Bandapparat geschwächt und besteht die Gefahr, dass damit die von den Wirbelkörpern auf die Zervikalprothese 1 ausgeübte Pressung gering ist, so kann es sich empfehlen, die Rippe 18 mit einer Durchbrechung 19, wie sie als gestrichelte Linie in Fig. 2 dargestellt ist, zu versehen. Die Durchbrechung 19 erlaubt es Knochenmaterial, nach der Implantation der Verankerungsplatten in den jeweiligen Wirbelkörper, durch diese Durchbrechung 19 zu wachsen. Damit wird die Verankerungsplatte an dem Wirbelkörper in der Weise fixiert, dass sie sich nicht von dem Wirbelkörper abheben kann.

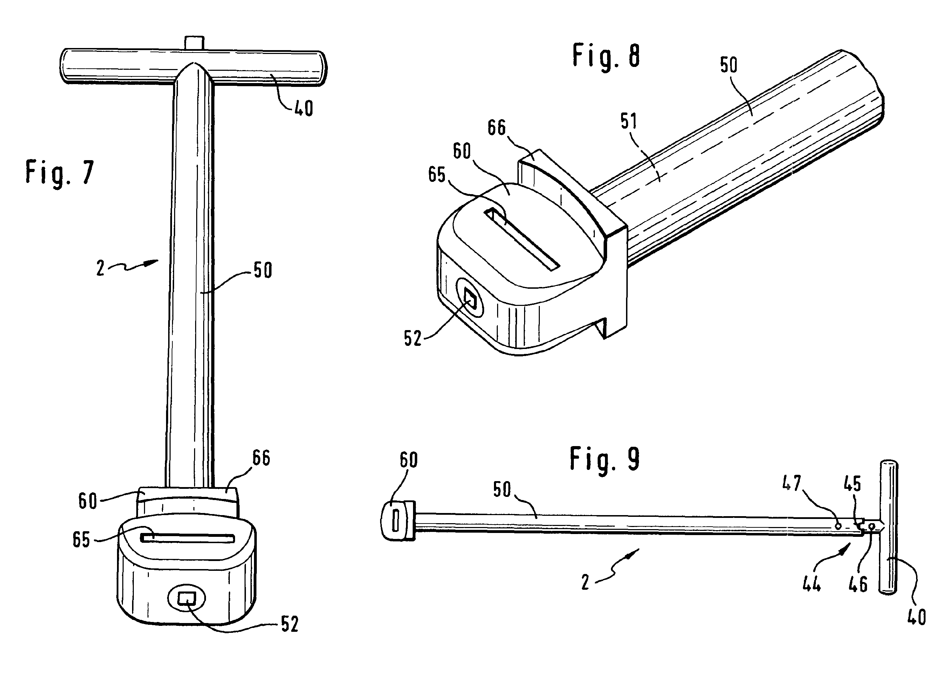

Die Rippe 18 weist eine Höhe von 1,5 mm auf. Sie soll, wie bereits ausgeführt, nicht selbstschneidend sein. Es ist daher erforderlich, bei der Präparation der Implantationsstelle eine entsprechende Ausnehmung in die entsprechenden Zwischenwirbelflächen einzuarbeiten. Dazu dient das in den Figuren 3 bis 9 dargestellte Instrument. Das Instrument ist in seiner Gesamtheit mit dem Bezugszeichen 2 bezeichnet. Es umfasst einen Handgriff 40, einen Stiel 50 sowie ein Kopfstück 60. Das Kopfstück 60 fungiert als Probestück und weist die Kontur und die Abmessungen der zu implantierenden zervikalprothese 1 auf. An dem anterioren Rand des Kopfstücks 60 ist ein Flansch 66 entsprechend dem Flansch 16 der Zervikalprothese 1 vorgesehen. Das Instrument kann damit als Probierlehre für die zu implantierende Zervikalprothese 1 dienen.

In dem Kopfstück 60 ist eine Messerscheibe 7 als Räumelement angeordnet. Dazu weist das Kopfstück 60 einen Schlitz 65 auf, der sich über die gesamte Höhe des Kopfstücks von der Oberseite 63 bis auf die Unterseite des Kopfstücks 60 erstreckt. Die Messerscheibe ist als Doppelfinnenmesser mit zwei Paaren von gegenüberliegenden Finnen ausgeführt. Das erste Finnenpaar sind Vorschneidfinnen 72, die jeweils eine in Umfangsrichtung wirkende Schneidkante aufweisen. Das zweite Finnenpaar sind zwei Hauptschneidfinnen 71, die einander gegenüberliegen und eine in die gleiche Richtung wie bei den Vorschneidfinnen 72 weisende Schneidkante aufweisen, die aber in Schneidrichtung gesehen winkelmäßig zurückversetzt ist, um einen Betrag von etwa 35 Grad. Die Messerscheibe 7 weist in ihrer Mitte eine quadratische Antriebsöffnung 73 auf.

An dem Handgriff 40 ist T-förmig eine Übertragungswelle 51 angeordnet, die durch den als Hohlzylinder ausgeführten Stiel 50 in das Kopfstück 60 führt. An ihrem vom Handgriff 40 entfernten Ende ist die Übertragungswelle 51 mit einem Mitnehmer-Vierkant 52 versehen. Die Übertragungswelle 51 ist an dem Stiel 50 drehbeweglich und längsbeweglich geführt. An dem griffseitigen Ende des Stiels 50 ist eine Indexeinrichtung 45, 46 angeordnet. Sie umfasst eine Ausnehmung 45 am Rand des Stiels 50 und einen Markierungsstift 46 am griffseitigen Ende der Übertragungswelle 51. In einer Ruheposition ist der Handgriff 40 mit der Übertragungswelle 51 so gedreht und in den Stiel 50 geschoben, dass der Markierungsstift 46 in der Ausnehmung 45 liegt. Zum Bewegen des Handgriffs 40 in seiner Arbeitsposition wird der Handgriff 40 mit der Übertragungswelle 51 ein Stück von dem Rand des Stiels 50 weggezogen, bis der Markierungsstift 46 frei von der Ausnehmung 45 ist und der Handgriff 40 mit der Übertragungswelle 51 verdreht werden kann. Der Mitnehmer-Vierkant 52 an dem grifffernen Ende der Übertragungswelle 51 ist mindestens über die Strecke, über die der Griff zum Freikommen des Markierungsstifts 46 aus der Ausnehmung 45 gezogen werden muss, als Vierkant ausgebildet. Dadurch ist sichergestellt, dass der Vierkant sich stets über den Bereich des Schlitzes 65 des Kopfstücks 60 erstreckt, und zwar unabhängig davon, ob sich der Griff 40 in seiner Ruheposition oder in seiner verdrehten Arbeitsposition befindet.

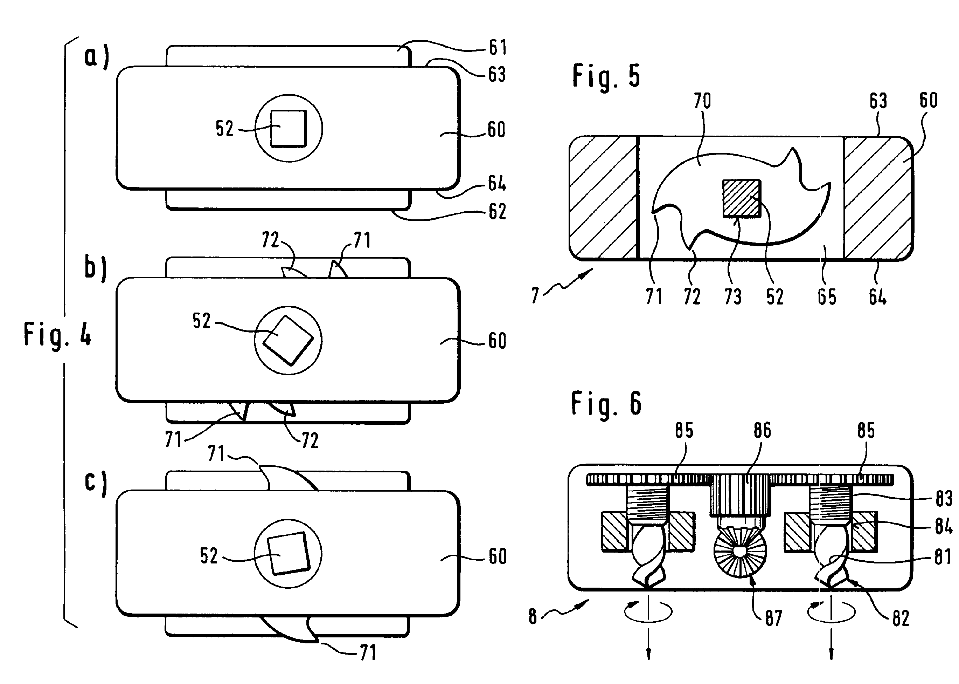

Die Funktionsweise der Messerscheibe 7 ist in Fig. 4 näher dargestellt. In Fig. 4 a ist die Ruheposition gezeigt. Die Messerscheibe 7 befindet sich in der Stellung, in der sie auch in Fig. 3 dargestellt ist. Die Schneidfinnen 71, 72 sind versenkt. Das Instrument kann in dieser Ruheposition in den Implantationsraum eingeschoben oder aus ihm herausgezogen werden. In Fig. 4 b und c sind Arbeitspositionen dargestellt. Zum Erreichen dieser Position wird der Handgriff 40 so weit herausgezogen, dass der Markierungsstift 46 frei von der Ausnehmung 45 an dem Stiel 50 ist. Der Handgriff 40 kann dann in der Richtung bewegt werden, in der die Schneidkanten der Schneidfinnen 71, 72 weisen. Durch die Drehung der Messerscheibe 7 bewegen sich die Schneidfinnen 71, 72 auf einer kreisförmigen Bahn. Zuerst verlassen dabei die Vorschneidfinnen 72 den Schlitz 65 des Kopfstücks 60 und schneiden einen ersten, niedrigen Schlitz in die angrenzende Fläche des Wirbelkörpers. Die Vorschneidfinnen sind so beschaffen, dass sie den vergleichsweise harten Rand des Wirbelkörpers durchbrechen. Danach bewegen sich die Hauptschneidfinnen 71 aus dem Schlitz 65 hervor und schneiden einen größeren Schlitz in dem gegenüber dem Rand weicheren Knochenmaterial des Wirbelkörpers. Die Drehung erfolgt so weit, dass die Hauptschneidfinnen 71 auf der gegenüberliegenden Seite wieder beginnen, in den Schlitz 65 des Kopfstücks 60 einzutauchen. Gewünschtenfalls kann der Vorgang wiederholt werden. Aufgrund der symmetrischen Gestaltung der Messerscheibe 7 werden die Schlitze in dem oberen und unteren Wirbelkörper gleichzeitig geschnitten. Wenn dies zur Verringerung der Betätigungskräfte nicht gewünscht ist, so können die Finnen entweder nur einseitig vorgesehen sein oder sie sind mit einem von 180 Grad abweichen Winkelversatz angeordnet, so dass zuerst ein Satz von Vorschneid- und Hauptschneidfinnen 71, 72 sich aus dem Schlitz 65 erhebt, während der andere erst später bei der Drehung folgt.

In Fig. 5 ist ein anderes Ausführungsbeispiel für ein Räumelement dargestellt. Es handelt sich um eine kombinierte Bohr-/Fräseinrichtung 8. Sie umfasst zwei Bohrer 82, die quer zur Richtung des Stiels 50 in dem Kopfstück 60 angeordnet sind. Die Bohrer 82 weisen in ihrem unteren Abschnitt Schneidzüge 81 auf. In ihrem oberen Abschnitt sind sie mit einem Außengewinde 83 versehen. Dieses ist geführt in einem fest in dem Kopfstück 60 angeordneten Gegengewinde 84. An dem oberen Ende des Bohrers 82 ist ein getriebenes Zahnrad 85 angeordnet. Es kämmt mit einem Treibrad 86, das wiederum über einen Winkeltrieb 87 von der Übertragungswelle 51 angetrieben ist. Das Treibrad 86 weist eine größere Stärke als das getriebene Rad 85 auf. Vorzugsweise entspricht seine Stärke dem vorgesehenen Bohrhub, d.h. der Tiefe der mit den Bohrern 82 zu erzeugenden Ausnehmungen. Auf der gegenüberliegenden Seite ist eine Bohreranordnung spiegelbildlich angeordnet. Die Bohr-/Fräseinrichtung 8 funktioniert wie folgt. Mittels eines vorzugsweisen maschinellen Antriebs wird die Übertragungswelle 51 in Drehungen versetzt, wodurch über den Winkeltrieb 87 das Treibrad 86 ebenfalls dreht. In ihrer Ruheposition befinden sich die Bohrer in der in Fig. 5 dargestellten Stellung, wobei das getriebene Rad 85 in das Treibrad 86 an dessen oberen Rand eingreift. Durch die Drehung des Treibrads 86 wird auch das getriebene Rad 85 gedreht, wodurch der Bohrer 82 in Drehbewegung versetzt wird. Aufgrund der Drehbewegung schraubt sich der Bohrer 82 mit seinem Gewinde 83 in das Außengewinde 84 hinein, wodurch der Bohrer 82 nach unten bewegt wird. Der Bohrer 82 arbeitet sich dadurch mit seinen Schneidzügen 81 in das Knochenmaterial des Wirbelkörpers ein. Aufgrund der nach unten gerichteten Schubbewegung des Bohrers 82 bewegt sich auch das getriebene Rad 85 nach unten, wobei es stets im Eingriff in das Treibrad 86 bleibt. Letzteres weist dazu eine Stärke auf, die mindestens so groß wie der Hub des Bohrers 82 ist. Ist die gewünschte Tiefe der Ausnehmungen erreicht, so wird durch Umkehren der Drehrichtung erreicht, dass die Bohrer 82 wieder in ihre Ruheposition fahren.

Die Rippe 18 kann bei einer alternativen Ausführungsform auch in Segmente 18' geteilt sein (siehe Fig. 3). Die Segmentierung hat den Vorteil, dass durch die dabei entstehenden Seitenflächen eine zusätzliche Sicherung der Prothese gegenüber unerwünschten Verschiebungen in der Transversalachse verhindert wird. Die Rippe 18 kann in zwei oder drei Segmente 18' unterteilt sein, wie durch die gestrichelte Linie in Fig. 3 angedeutet ist. Zur Verbesserung des Halts an dem Wirbelkörper kann vorgesehen sein, die Ausnehmung in dem Wirbelkörper zur Aufnahme der Rippensegmente 18' nicht durchgängig auszuführen, sondern ebenfalls zu segmentieren. Nähere Ausführungen dazu sind im Bezug auf die Bohr-/Fräseinrichtung 8 gegeben.

Das Verfahren zur Implantation der erfindungsgemäßen Prothesen unter Verwendung des erfindungsgemäßen Werkzeugs lässt sich wie folgt darstellen. In einem ersten Schritt werden die aneinandergrenzenden Wirbelkörper, in deren Zwischenraum die Zervikalprothese 1 zu implantieren ist, zur Aufnahme einer Spreizvorrichtung 91 vorbereitet. Dies geschieht, indem die Schenkel 92, 93 des Spreizwerkzeugs 91 mittels einer Schraubbefestigung an der anterioren Seite der beiden Wirbelkörper befestigt wird. Das Spreizwerkzeug 91 ist in der Weise abgewinkelt ausgeführt, dass der unmittelbare Zugangsbereich von anterior in den Zwischenraum frei bleibt. Nach dem Spreizen der Wirbel auf die vorgesehene Entfernung wird der Zwischenraum zur Aufnahme der Zervikalprothese 1 vorbereitet. Dies geschieht durch Räumen von überschüssigem Knochenmaterial, um eine geeignete Anlagefläche für die Verankerungsplatten 11, 12 und für den Flansch 16 herzustellen (siehe Fig. 10). Nachdem die Implantationsstelle soweit vorbereitet wurde, wird das erfindungsgemäße Instrument 2 angesetzt. Das Kopfstück 60 wird in den vorbereiteten Zwischenwirbelraum geschoben. Durch Betätigen des Handgriffs 40 wird die Messerscheibe 7 betätigt, wobei die Schneidfinnen 71, 72 eine Aufnahme für die Rippe 18 in den kranial und kaudal benachbarten Wirbelkörper schneiden. Danach wird die Messerscheibe 7 wieder in ihre Ruheposition geführt und das Instrument 2 kann herausgezogen werden. Damit ist die Vorbereitung vervollständigt. Die Zervikalprothese 1 kann nun eingesetzt werden, wobei gegebenenfalls die Wirbelkörper mittels des Spreizwerkzeugs 91 etwas überspreizt werden, um ausreichend Raum zum Einführen der Rippen 18 in die Ausnehmungen zu haben. Nach dem Abnehmen des Spreizwerkzeugs 91 ist die Implantation vollständig.

## Patentansprüche

1. Zervikale Zwischenwirbelprothese umfassend eine untere und eine obere Verankerungsplatte (11, 12), die mit jeweils einer Verankerungsplattenoberfläche zur Anlage an einem benachbarten Wirbelkörper ausgebildet sind, sowie einen dazwischen angeordneten Prothesenkern (10), der eine gelenkige Verbindung zwischen den Verankerungsplatten (11, 12) bildet,
**dadurch gekennzeichnet, dass**
mindestens eine der beiden Verankerungsplattenoberflächen (11, 12) zum Wirbelkörper einen rippenartigen Vorsprung (18) zum formschlüssigen Eingriff in den Wirbelkörper quer zur AP-Richtung aufweist.

2. Zervikale Zwischenwirbelprothese nach Anspruch 1,
**dadurch gekennzeichnet, dass**
der Vorsprung (18) außerhalb eines Randbereichs der Verankerungsplattenoberfläche (11, 12) angeordnet ist.

3. Zervikale Zwischenwirbelprothese nach Anspruch 2,
**dadurch gekennzeichnet, dass**
der Vorsprung posterior von der Mitte versetzt angeordnet ist, vorzugsweise in einem Bereich zwischen 3/5 und 3/4 der Erstreckung in AP-Richtung.

4. Zervikale Zwischenwirbelprothese nach einem der Ansprüche 1 bis 3,
**dadurch gekennzeichnet, dass**
der Vorsprung (18) eine Höhe von 0,3 bis 5,0 mm über dem Niveau der Verankerungsplattenoberfläche (11, 12) aufweist.

5. Zervikale Zwischenwirbelprothese nach einem der vorhergehenden Ansprüche,
**dadurch gekennzeichnet, dass**
der Vorsprung (18) ein Kugelabschnitt ist.

6. Zervikale Zwischenwirbelprothese nach einem der vorhergehenden Ansprüche,
**dadurch gekennzeichnet, dass**
der Vorsprung (18) in zwei oder mehr Segmente (18') mit dazwischen liegender Lücke unterteilt ist.

7. Zervikale Zwischenwirbelprothese nach Anspruch 6,
**dadurch gekennzeichnet, dass**
die Segmente (18') bolzenartig ausgeführt sind.

8. Instrument zum Implantieren einer zervikalen Zwischenwirbelprothese (1), die zwei Verankerungsplatten (11, 12) und einen dazwischen angeordneten Prothesenkern (10) umfasst, mit einem Griff (40), einem Stiel (50) und einem Kopfstück (60) das an einem vom Griff (40) entfernten Ende angeordnet ist und dessen Abmessungen so gewählt sind, dass es in den zur Aufnahme der Zwischenwirbelprothese (1) geschaffenen Raum zwischen benachbarten Wirbelkörper einschiebbar ist,
**dadurch gekennzeichnet, dass**
das Kopfstück (60) ein Räumelement (7, 8) zum Schaffen einer Ausnehmung in kranial-kaudaler Richtung in den Wirbelkörpern aufweist und eine Betätigungseinrichtung (40, 51, 52) für das Räumelement (7, 8) vorgesehen ist, das zwischen einer Ruheposition, in der es in dem Kopfstück (60) versenkt ist, und einer Arbeitsposition, in der es quer zum Stiel (50) aus dem Kopfstück (60) herausragt, beweglich ist.

9. Instrument nach Anspruch 8,
**dadurch gekennzeichnet, dass**
das Räumelement eine Messerscheibe (7) ist.

10. Instrument nach Anspruch 8,
**dadurch gekennzeichnet, dass**
die Messerscheibe (7) mindestens ein Paar über dem Umfang versetzt angeordnete Schneidfinnen (71, 72) aufweist.

11. Instrument nach Anspruch 10,
**dadurch gekennzeichnet, dass**
die Schneidfinnen (71, 72) unterschiedliche Höhen aufweisen.

12. Instrument nach Anspruch 10 oder 11,
**dadurch gekennzeichnet, dass**
die Schneidfinnen (71, 72) paarig gegenüberliegend angeordnet sind.

13. Instrument nach Anspruch 8,
**dadurch gekennzeichnet, dass**
das Räumelement (8) ein Bohrer (82) ist.

14. Instrument nach Anspruch 13,
**dadurch gekennzeichnet, dass**
ein Schub-/Schraubtrieb (85, 83, 84) zur Betätigung des Bohrers (82) vorgesehen ist.

15. Instrument nach Anspruch 13 oder 14,
**dadurch gekennzeichnet, dass**
mindestens zwei Bohrer (82) quer zum Stiel (50) angeordnet sind.

16. Instrument nach einem der Ansprüche 13 bis 15,
**dadurch gekennzeichnet, dass**
das Räumelement (8) einen Kugelfräserabschnitt ausweist.

17. Instrument nach einem der Ansprüche 8 bis 16,
**dadurch gekennzeichnet, dass**
das Räumelement (8) entlang einer Führung (84) längsbeweglich ist.

18. Instrument nach einem der Ansprüche 8 bis 17,
**dadurch gekennzeichnet, dass**
das Betätigungselement einen Handgriff (40) und eine Übertragungswelle (51) umfasst.

19. Instrument nach einem der Ansprüche 8 bis 18,
**dadurch gekennzeichnet, dass**
das Betätigungselement eine Drehantriebskupplung aufweist.
